(19) Europäisches Patentamt
European Patent Office
Office européen des brevets

(11) Veröffentlichungsnummer: **0 003 028**
**B1**

(12) # EUROPÄISCHE PATENTSCHRIFT

(45) Veröffentlichungstag der Patentschrift: **05.08.81**

(51) Int. Cl.³: **C 07 C 103/52,** A 61 K 37/02

(21) Anmeldenummer: **78101625.8**

(22) Anmeldetag: **09.12.78**

(54) **Beta h-Endorphin-Analoge, diese enthaltende Präparate und deren Herstellung.**

(30) Priorität: **16.01.78 US 869555**

(43) Veröffentlichungstag der Anmeldung: **25.07.79 Patentblatt 79/15**

(45) Bekanntmachung des Hinweises auf die Patenterteilung: **05.08.81 Patentblatt 81/31**

(84) Benannte Vertragsstaaten: **IT**

(56) Entgegenhaltungen:
**US-A-4 081 434**

**Unlisted Drugs, 1977, Special Library Association, März 1977, Vol. 29, Nr. 3, Seite 44d**
***Zusammenfassung***

**Unlisted Drugs, 1977, Special Library Association, Mai 1977, Vol. 29, Nr. 5**
***Zusammenfassung***

(73) Patentinhaber: **F. HOFFMANN-LA ROCHE & CO. Aktiengesellschaft, CH-4002 Basel (CH)**

(72) Erfinder: **Li, Choh Hao, 901 Arlington Avenue, Berkeley Kalifornien (US)**

(74) Vertreter: **Mezger, Wolfgang, Dr. et al, Grenzacherstrasse 124 Postfach 601, CH-4002 Basel (CH)**

EP 0 003 028 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Beta$_h$-Endorphin-Analoge, diese enthaltende Präparate und deren Herstellung

Die Isolierung von $\beta$-Endorphinen aus Gehirn- und Hypophysen-Extrakten von Säugern wie auch ihre Synthese unter Verwendung von Methoden der Festphasentechnik ist aus der Literatur bekannt (z. B. Li und Chung, Proc. Natl. Acad. Sci. USA 73, 1145 [1976] und US-Patent Nr. 4038222). Das $\beta$-Endorphin wurde dann zunächst in den Positionen 1–5 der Aminosäuresequenz modifiziert, doch erreichten die bis jetzt publizierten Analogen – mit Ausnahme des [D-Ala$^2$]-$\beta$-Endorphins – nicht die Aktivität der Grundverbindung (Yamashiro et al., Int. J. Pept. Prot. Res. 10, 159 [1977]).

Die vorliegende Erfindung betrifft neue Analoge des menschlichen $\beta$-Endorphins ($\beta_h$-Endorphin) der Formel

$$[X, Phe^{27}, Gly^{31}]\text{-}\beta_h\text{-Endorphin} \qquad I$$

worin X null, Ala$^{6,7}$, Nle$^5$ oder D-Thr$^2$ ist, und deren physiologisch verträgliche Säureadditionssalze, Verfahren zu deren Herstellung und pharmazeutische Präparate auf der Basis dieser Verbindungen.

Die Formel I umfasst die folgenden Verbindungen:

[Phe$^{27}$, Gly$^{31}$]-$\beta_h$-Endorphin,
[Ala$^{6,7}$, Phe$^{27}$, Gly$^{31}$]-$\beta_h$-Endorphin,
[Nle$^5$, Phe$^{27}$, Gly$^{31}$]-$\beta_h$-Endorphin und
[D-Thr$^2$, Phe$^{27}$, Gly$^{31}$]-$\beta_h$-Endorphin.

Die erfindungsgemässen Verbindungen können nach aus der Peptidchemie allgemein bekannten Methoden hergestellt werden. Dies schliesst ein, dass man in der letzten Stufe einer konventionellen Fest- oder Flüssigphasen-Synthese den festen Träger und/oder die Schutzgruppen des geschützten $\beta$-Endorphin-Analogen (das im Falle der Festphasensynthese an den festen Träger gebunden ist) in an sich bekannter Weise abspaltet und gewünschtenfalls die erhaltene Verbindung I in ein physiologisch verträgliches Säureadditionssalz überführt. In einem bevorzugten erfindungsgemässen Verfahren können die neuen $\beta$-Endorphin-Analogen nach der Festphasenmethode von Merrifield (J. Am. Chem. Soc. 85, 2149 [1963]), besonders mit den Modifizierungen von Yamashiro und Li (Proc. Natl. Acad. Sci. USA 71, 4945 [1974]), hergestellt werden.

In einer geeigneten Festphasensynthese wird das Carboxy-terminale Glycin mit geschützter Aminogruppe (Boc-Glycin) an ein in der Peptid-Festphasensynthese üblicherweise verwendetes Trägerharz, beispielsweise an ein chlormethyliertes Polystyrolharz, das mit 1–2% Divinylbenzol (w/w) vernetzt ist, gekoppelt. Das die Boc-geschützte Aminosäure tragende Harz wird dann alternierend mit 55%iger (v/v) Trifluoressigsäure in Methylenchlorid zwecks Abspaltung der Schutzgruppe behandelt, mit Diisopropyläthylamin neutralisiert und mit dem symmetrischen Anhydrid der nächsten Boc-Aminosäure der Sequenz umgesetzt. Nachdem auf diese Weise die letzte geschützte Aminosäure angeknüpft wurde, wird das erhaltene geschützte, an das Trägermaterial gekoppelte Peptid mit flüssigem HF in an sich bekannter Weise behandelt, wodurch das freie Peptid erhalten wird. Das Rohprodukt wird dann durch Chromatographie an Carboxymethylcellulose und Verteilungschromatographie an Sephadex® G-50 gereinigt.

Die Charakterisierung der synthetisierten Peptide erfolgte durch Aminosäureanalyse nach saurer Hydrolyse und enzymatischem Abbau, durch Papierelektrophorese und Dünnschichtchromatographie.

Die erfindungsgemässen Verbindungen sind starke Opiatagonisten und können als Analgetika, Narkotika-Antagonisten und Mittel gegen Diarrhöe verwendet werden.

Die erfindungsgemässen Verbindungen können als pharmazeutische Präparate mit direkter oder verzögerter Freigabe des Wirkstoffs in Mischung mit einem für die enterale oder parenterale Applikation geeigneten organischen oder anorganischen inerten Trägermaterial, wie z. B. Wasser, Gelatine, Gummi arabicum, Milchzucker, Stärke, Magnesiumstearat, Talk, pflanzlichen Ölen, Polyalkylenglykolen, Vaseline, usw. verwendet werden. Die pharmazeutischen Präparate können in fester Form (z. B. als Tabletten, Dragées, Kapseln) oder in flüssiger Form (z. B. als Lösungen, Suspensionen oder Emulsionen) vorliegen. Gegebenenfalls sind sie sterilisiert und bzw. oder enthalten weitere Hilfsstoffe, wie Konservierungs-, Stabilisierungs-, Netz- oder Emulgiermittel, Mittel zur geschmacklichen Verbesserung, Salze zur Veränderung des osmotischen Druckes oder Puffersubstanzen. Die Herstellung der pharmazeutischen Präparate kann in der jedem Fachmann geläufigen Weise erfolgen.

Die erfindungsgemässen Verbindungen werden zweckmässigerweise parenteral, vorzugsweise intravenös (iv), bei einer Dosierung von ca. 1–50 mg, verabreicht. Für die parenterale Verabreichung eignet sich besonders eine aus der gereinigten und lyophilisierten Wirksubstanz durch Zusatz von destilliertem, sterilem Wasser oder physiologischer Kochsalzlösung rekonstituierte Lösung.

Als physiologisch verträgliche Säureadditionssalze der erfindungsgemässen Verbindungen I kommen solche in Frage, die sich von anorganischen und organischen Säuren wie Schwefelsäure, Phosphorsäure, Chlorwasserstoffsäure, Bromwasserstoffsäure, Jodwasserstoffsäure, Salpetersäure, Sulfamsäure, Zitronensäure, Milchsäure, Brenztraubensäure, Oxalsäure, Maleinsäure, Bernsteinsäure, Weinsäure, Zimtsäure, Essigsäure, Trifluoressigsäure, Benzoesäure, Salicylsäure, Gluconsäure, Ascorbinsäure und ähnlichen ableiten.

Beispiel

Die vier Peptide [$Phe^{27}$, $Gly^{31}$]-$\beta_h$-Endorphin (I), [$Ala^{6,7}$, $Phe^{27}$, $Gly^{31}$]-$\beta_h$-Endorphin (II), [$Nle^{5}$, $Phe^{27}$, $Gly^{31}$]-$\beta_h$-Endorphin (III) und [D-$Thr^2$, $Phe^{27}$, $Gly^{31}$]-$\beta_h$-Endorphin (IV) wurden nach verbesserten, an sich bekannten Festphasen-Methoden synthetisiert.

Herstellung der geschützten, an das Trägerharz gekoppelten Peptide I–IV

Das Boc-glycyl-Copolystyrol-Divinylbenzol-Harz (1,51 g, 0,60 mMol Glycin) wurde den folgenden Operationen unterworfen:

1. Waschen mit Methylenchlorid, 4× mit 20 ml;
2. Waschen mit 55%iger (v/v) Trifluoressigsäure in Methylenchlorid, 1× mit 20 ml;
3. Umsetzung mit 20 ml 55%iger (v/v) Trifluoressigsäure in Methylenchlorid während 15 Minuten (Entfernung der Boc-Schutzgruppe);
4. Waschen 2× mit 20 ml Methylenchlorid;
5. Waschen mit Dioxan/Methylenchlorid (1:2, v/v) 3× mit 20 ml;
6. Waschen 2× mit 20 ml Methylenchlorid;
7. Umsetzung mit 20 ml 5%igem (v/v) Diisopropylenäthylamin in Methylenchlorid während 2 Minuten;
8. Waschen 2× mit 20 ml Methylenchlorid;
9. Umsetzung mit 20 ml 5%igem (v/v) Diisopropyläthylamin in Methylenchlorid während 2 Minuten;
10. Waschen 5× mit 20 ml Methylenchlorid;
11. Umsetzung mit 1,8 mMol des symmetrischen Anhydrids der jeweiligen Boc-Aminosäure in 13 ml Methylenchlorid während 20 Minuten;
12. Zusatz von 0,3 mMol N-Methylmorpholin in 2 ml Methylenchlorid und Schütteln während 20 Minuten;
13. Waschen 3× mit je 20 ml Methylenchlorid und schliesslich
14. Waschen mit Äthanol/Methylenchlorid (1:2, v/v), 3× mit je 20 ml.

Nach Kopplung der letzten Aminosäure an das Peptidharz wurde das Produkt den Operationen 1–8 unterworfen, mit Äthanol gewaschen und schliesslich getrocknet.

Die $\alpha$-Aminogruppen aller Aminosäuren wurden durch die Boc-Gruppe geschützt, während die Hydroxygruppen von Ser und Thr durch eine O-Benzylgruppe, die Carboxylgruppe von Glu als Benzylester, die Aminogruppe von Lys durch o-BrZ und die Hydroxygruppe von Tyr durch Z geschützt waren. Die symmetrischen Anhydride der Boc-Aminosäuren wurden nach der von Blake und Li (Int. J. Pept. Prot. Res. 7, 495 [1975]) beschriebenen Methode hergestellt und Boc-Asparagin wurde in Gegenwart von 1-Hydroxybenzotriazol (ebenfalls dort beschrieben) an das Peptidharz gekoppelt.

Isolierung der Peptide I–IV

Das Peptid I-Harz (302 mg, 0,060 mMol) wurde mit 1 ml Anisol und 8 ml flüssigem Fluorwasserstoff während einer Stunde bei 0° C behandelt. Bei 0° C wurde der Fluorwasserstoff abgezogen, dem Rückstand wurden 40 ml kaltes Äthylacetat zugesetzt und das Gemisch 10 Minuten bei Raumtemperatur gerührt. Dann wurde filtriert, der Niederschlag mit Äthylacetat gewaschen und an der Luft getrocknet. Das Peptid wurde aus dem Rückstand durch Rühren mit 6 ml 0,5n Essigsäure und anschliessende Filtration extrahiert. Das Filtrat wurde an Sephadex® G-10 chromatographiert. Lyophilisation der Hauptfraktion lieferte 97 mg rohes Peptid I. Chromatographie des Rohproduktes an Carboxymethylcellulose wie von Li et al. (Biochem. Biophys. Res. Commun. 71, 19 [1976]) beschrieben, lieferte 41,2 mg Peptid I. Weitere Reinigung erfolgte durch Verteilungschromatographie an einer Sephadex® G-50-Säule (1,9×44 cm) mit n-Butanol/Pyridin/0,1% Essigsäure (5:3:11, v/v/v). Das dem Hauptpeak mit einem $R_f$-Wert von 0,23 zuzuordnende Material wurde vereinigt. Es wurde zunächst mit einer gleichgrossen Menge Wasser verdünnt, dann unter vermindertem Druck auf etwa 10 ml eingeengt und die konzentrierte Lösung lyophilisiert. Das erhaltene Produkt wurde nochmals in verdünnter Essigsäure aufgelöst und lyophilisiert und lieferte 29,2 mg Peptid I (14,4%, bezogen auf Boc-glycyl-Harz). Die Peptide II, III und IV wurden in Ausbeuten von 10%, 28% und 21% erhalten.

Die Papierelektrophorese des Peptids I bei pH 3,7 und 6,7 (400 Volt, $3^1/_2$ Stunden) lieferte jeweils einen einzigen Ninhydrin-positiven und Chlor-positiven Fleck mit $R_f^{Lys}$ 0,56 bzw. 0,38. Dünnschichtchromatographie auf Kieselgel mit n-Butanol/Pyridin/Essigsäure/Wasser (5:5:1:4, v/v/v/v) lieferte einen einzigen Ninhydrin- und Chlor-positiven Fleck bei $R_f$ 0,51.

Zwecks enzymatischen Abbaus wurde eine Lösung von jeweils 0,7 mg des Peptids mit 14 $\mu$g Chymotrypsin und 14 $\mu$g Trypsin in 0,15 ml Tris-Puffer (pH 8,5, 0,01 M $Mg^{2+}$) 24 Stunden bei 37° C inkubiert. Die Lösung wurde 15 Minuten in kochendem Wasser erhitzt, auf Raumtemperatur abgekühlt und weitere 44 Stunden mit 28 $\mu$g Leucinaminopeptidase bei 37° C inkubiert. Schliesslich wurde die Lösung in einem Aminosäureanalysator analysiert. Sowohl die nach enzymatischem Abbau als auch nach saurer Hydrolyse erhaltenen Ergebnisse bestätigten die für die Peptide I–IV vorstehend angegebenen Formeln.

Bioassay

Der in vitro-Assay auf Opiataktivität wurde nach der Meerschweinchen-Ileum-Methode von Kosterlitz et al. (Brit. J. Pharmacol. 39, 398 [1970]) und der in vivo-Assay an Mäusen nach der Tail-Flick-Methode von D'Amour und Smith (J. Pharmacol. Exp. Therap. 72, 74 [1941]) durchgeführt. Die experimentelle Prozedur und die Auswertung wurden von Loh et al. (Proc. Natl. Acad. Sci. 73, 2895 [1976] und Tseng et al. (Nature 263, 239 [1976]) beschrieben.

Die Testergebnisse der Peptide I–IV sind in den folgenden Tabellen 1 und 2 zusammengefasst.

Tabelle 1
Opiat-Aktivität von synthetischen $\beta$-Endorphin-Analogen im Meerschweinchen-Ileum-Assay

| Verbindung | $IC_{50}$ [Mol/l] | Relative Aktivität[a] |
|---|---|---|
| $\beta_h$-Endorphin | $4{,}5\times10^{-8}$ | 100 |
| [$Phe^{27}$, $Gly^{31}$]-$\beta_h$-Endorphin (I) | $3{,}5\times10^{-8}$ | 128 |
| [$Ala^{6,7}$, $Phe^{27}$, $Gly^{31}$]-$\beta_h$-Endorphin (II) | $3{,}8\times10^{-8}$ | 118 |
| [$Nle^{5}$, $Phe^{27}$, $Gly^{31}$]-$\beta_h$-Endorphin (III) | $3{,}8\times10^{-8}$ | 118 |
| [D-$Thr^{2}$, $Phe^{27}$, $Gly^{31}$]-$\beta_h$-Endorphin (IV) | $3{,}3\times10^{-8}$ | 136 |

[a] bezogen auf $IC_{50}$ von $\beta_h$-Endorphin als 100%.

Tabelle 2
Analgetische Wirkung von synthetischen $\beta_h$-Endorphin-Analogen bei Mäusen

| Verbindung | icv $AD_{50}$ [$\mu$g/Maus][a] | relative Aktivität[b] | iv $AD_{50}$ [mg/kg][a] | relative Aktivität[b] |
|---|---|---|---|---|
| $\beta_h$-Endorphin | 0,11 (0,07–0,17) | 100 | 11,4 (6,4–19,6) | 100 |
| [$Phe^{27}$, $Gly^{31}$]-$\beta_h$-Endorphin (I) | 0,09 (0,08–0,11) | 119 | 7,5 (3,4–16,5) | 148 |
| [$Ala^{6,7}$, $Phe^{27}$, $Gly^{31}$]-$\beta_h$-Endorphin (II) | 0,46 (0,28–0,77) | 23 | 22,1 (14,3–34,3) | 49 |
| [$Nle^{5}$, $Phe^{27}$, $Gly^{31}$]-$\beta_h$-Endorphin (III) | 0,31 (0,14–0,72) | 35 | ca. 17,0 | 65 |
| [D-$Thr^{2}$, $Phe^{27}$, $Gly^{31}$]-$\beta_h$-Endorphin (IV) | 0,53 (0,24–1,16) | 21 | ca. 32,0 | 32 |

[a] Werte in Klammern = 95%-Vertrauensgrenzen
[b] Relative Aktivitäten auf molarer Basis bezogen auf $AD_{50}$ von $\beta_h$-Endorphin als 100%.

Es ist festzuhalten, dass es nur eine geringe Korrelation zwischen den Ergebnissen aus dem in vitro-Assay nach der Meerschweinchen-Ileum-Methode und denen des in-vivo-Tests an der Maus nach intravenöser (iv) oder intracerebroventrikulärer (icv) Verabreichung gibt. Alle synthetischen Analogen des $\beta_h$-Endorphins sind etwas stärker wirksam als das $\beta_h$-Endorphin selbst im Meerschweinchen-Ileum-Assay und, mit Ausnahme des Peptids I, signifikant schlechter wirksam im in-vivo-Assay an der Maus. Die Dauer der analgetischen Wirkung scheint nach iv-Injektionen kürzer zu sein als nach icv-Injektionen. Das Peptid I scheint das erste synthetische Analoge des $\beta_h$-Endorphins mit gleicher Kettenlänge zu sein, das in vivo eine grössere analgetische Aktivität als das natürliche Peptid besitzt.

**Patentansprüche**

1. Analoge des $\beta_h$-Endorphins der Formel

[X, $Phe^{27}$, $Gly^{31}$]-$\beta_h$-Endorphin I

worin X null, $Ala^{6,7}$, $Nle^{5}$ oder D-$Thr^{2}$ ist, und deren physiologisch verträgliche Säureadditionssalze.

2. Verfahren zur Herstellung von Analogen des $\beta_h$-Endorphins der Formel

[X, $Phe^{27}$, $Gly^{31}$]-$\beta_h$-Endorphin I

worin X null, $Ala^{6,7}$, $Nle^{5}$ oder D-$Thr^{2}$ ist, und von deren physiologisch verträglichen Säureadditionssalzen, dadurch gekennzeichnet, dass man in der letzten Stufe einer konventionellen Fest- oder Flüssigphasen-Synthese den festen Träger und/oder die Schutzgruppen der geschützten $\beta_h$-Endorphin-Analogen in an sich bekannter Weise abspaltet und gewünschtenfalls die erhaltene Verbindung I in ein physiologisch verträgliches Säureadditionssalz überführt.

3. Pharmazeutische Präparate auf der Basis eines $\beta_h$-Endorphin-Analogen gemäss Anspruch 1.

**Claims**

1. Analogs of $\beta_h$-endorphin of the formula

[X, $Phe^{27}$, $Gly^{31}$]-$\beta_h$-endorphin I

wherein X is null, $Ala^{6,7}$, $Nle^{5}$ or D-$Thr^{2}$ and physiologically acceptable acid addition salts thereof.

2. A process for the preparation of $\beta_h$-endorphin analogs of the formula

[X, $Phe^{27}$, $Gly^{31}$]-$\beta_h$-endorphin I

wherein X is null, $Ala^{6,7}$, $Nle^{5}$ or D-$Thr^{2}$ and of physiologically acceptable acid addition salts thereof, characterized in that in the last step of a conventional solid or liquid phase synthesis the solid resin support and/or the protecting groups

of the protected $\beta_h$-endorphin analogs are cleaved off in a manner known per se and that, if desired, physiologically acceptable acid addition salts are formed.

3. Pharmaceutical preparations on the basis of a $\beta_h$-endorphin analog according to Claim 1.

**Revendications**

1. Les analogues de bêta$_h$-endorphine de la formule

(X, $Phe^{27}$, $Gly^{31}$)-$\beta_h$-endorphine I

dans laquelle X est nul, $Ala^{6,7}$, $Nle^5$ ou D-$Thr^2$ et leurs sels d'addition d'acide physiologiquement acceptables.

2. Un procédé pour la préparation d'analogues de bêta$_h$-endorphine de la formule

(X, $Phe^{27}$, $Gly^{31}$)-$\beta_h$-endorphine I

dans laquelle X est nul, $Ala^{6,7}$, $Nle^5$ ou D-$Thr^2$ et de leurs sels d'addition d'acide physiologiquement acceptables, caractérisé en ce que dans la dernière étape d'une synthèse classique en phase solide ou liquide le support de résine solide et/ou les groupes protecteurs des analogues de bêta$_h$-endorphine protégés sont éliminés d'une manière en elle-même connue et que, si on le désire, des sels d'addition d'acide physiologiquement acceptables sont formés.

3. Les compositions pharmaceutiques à base d'un analogue de bêta$_h$-endorphine selon la revendication 1.